# FASCICULE DE BREVET EUROPEEN

(11) **EP 1 456 189 B1**
(45) Date de publication et mention de la délivrance du brevet: **04.07.2007**
(21) Numéro de dépôt: 02805407.0
(22) Date de dépôt: 20.12.2002
(51) Int. Cl.: C07D 279/02, C07D 285/24, A61K 31/5415, A61K 31/549, A61P 25/28

(54) **DERIVES DE BENZOTHIA(DIA)ZINE ET LEUR UTILISATION COMME MODULATEURS AMPA**
BENZOTHIA(DIA)ZINDERIVATE UND IHRE VERWENDUNG ALS AMPA-MODULATOREN
BENZOTHIA(DIA)ZINE DERIVATIVES AND THEIR USE AS AMPA MODULATORS

(30) Priorité: 21.12.2001 FR 0116622
(43) Date de publication de la demande: 15.09.2004
(73) Titulaire: Les Laboratoires Servier, 92415 Courbevoie Cédex (FR)
(72) Inventeur: CORDI, Alex, F-92150 Suresnes (FR); DESOS, Patrice, F-92270 Bois Colombes (FR); LESTAGE, Pierre, F-78170 La Celle Saint Cloud (FR)
(86) Numéro de dépôt international: PCT/FR2002/004484
(87) Numéro de publication internationale: WO 2003/053947

(56) Documents cités:
- WO-A-01/40210
- WO-A-93/21170
- WO-A-98/12185
- WO-A-99/42456

## Description

La présente invention concerne de nouveaux dérivés de benzothiazine et de benzothiadiazine, leur procédé de préparation et les compositions pharmaceutiques qui les contiennent.

Il est désormais reconnu que les aminoacides excitateurs et tout particulièrement le glutamate, jouent un rôle crucial dans les processus physiologiques de plasticité neuronale et dans les mécanismes sous-tendant l'apprentissage et la mémoire. Des études pathophysiologiques ont indiqué clairement qu'un déficit de la neurotransmission glutamatergique est associé étroitement avec le développement de la maladie d'Alzheimer (Neuroscience and Biobehavioral reviews, 1992, 16, 13-24 ; Progress in Neurobiology, 1992, 39,517-545).

Par ailleurs, d'innombrables travaux ont démontré durant les dernières années l'existence de sous-types réceptoriels aux aminoacides excitateurs et de leurs interactions fonctionnelles (Molecular Neuropharmacology, 1992, 2, 15-31).

Parmi ces récepteurs, le récepteur à l'AMPA ("α-amino-3-hydroxy-5-methyl-4-isoxazole-propionic acid") apparaît être le plus impliqué dans les phénomènes d'excitabilité neuronale physiologique et notamment dans ceux impliqués dans les processus de mémorisation. Pour exemple, l'apprentissage a été montré comme étant associé à l'augmentation de la liaison de l'AMPA à son récepteur dans l'hippocampe, l'une des zones cérébrales essentielles aux processus mnémocognitifs. De même, les agents nootropes tels que l'aniracetam ont été décrits très récemment comme modulant positivement les récepteurs AMPA des cellules neuronales (Journal of Neurochemistry, 1992, 58, 1199-1204).

Dans la littérature, des composés de structure benzamide ont été décrits pour posséder ce même mécanisme d'action et pour améliorer les performances mnésiques (Synapse, 1993, 15, 326-329). Le composé BA 74, en particulier, est le plus actif parmi ces nouveaux agents pharmacologiques.

Enfin, le brevet EP 692 484 décrit un dérivé de benzothiadiazine possédant une activité facilitatrice sur le courant AMPA et la demande de brevet WO 99/42456 décrit entre autres certains dérivés de benzothiadiazine en tant que modulateurs des récepteurs AMPA.

Les dérivés de benzothiazine, objets de la présente invention, outre le fait qu'ils soient nouveaux, présentent, de manière surprenante, des activités pharmacologiques sur le courant AMPA nettement supérieures à celles des composés de structures proches décrits dans l'Art Antérieur. Ils sont utiles en tant que modulateurs AMPA pour le traitement ou la prévention des désordres mnémocognitifs associés à l'âge, aux syndromes anxieux ou dépressifs, aux maladies neurogénératives progressives, à la maladie d'Alzheimer, à la maladie de Pick, à la chorée d'Huntington, à la schizophrénie, aux séquelles des maladies neurodégénératives aiguës, aux séquelles de l'ischémie et aux séquelles de l'épilepsie.

Plus spécifiquement, la présente invention concerne les composés de formule **(I)**: 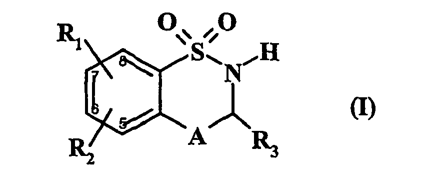 dans laquelle :
- **R₁**: représente un groupement hydroxy ou un groupement RCO-O-,
- **R₂**: représente un atome d'hydrogène, d'halogène, un groupement hydroxy ou un groupement R'CO-O,
- **R, R',**: identiques ou différents, représentent un groupement alkyle (C₁-C₆) linéaire ou ramifié éventuellement substitué par un groupement aryle, alkényle (C₂-C₆) linéaire ou ramifié éventuellement substitué par un groupement aryle , perhalogénoalkyle (C₁-C₆) linéaire ou ramifié, cycloalkyle (C₃-C₇), adamantyle, aryle ou hétéroaryle,
- **R₃**: représente un atome d'hydrogène, un groupement alkyle (C₁-C₆) linéaire ou ramifié ou un groupement cycloalkyle (C₃-C₇),
- **A**: représente un groupement CR₄R₅ ou un groupement NR₄,
- **R₄**: représente un atome d'hydrogène ou un groupement alkyle (C₁-C₆) linéaire ou ramifié
- **R₅**: représente un atome d'hydrogène ou d'halogène,
leurs isomères ainsi que leurs sels d'addition à un acide ou à une base pharmaceutiquement acceptable,
étant entendu que :
◆ R₁ représente un groupement RCO-O- lorsqu'il est situé en position 5 du composé de formule (I),
◆ par groupement aryle, on comprend groupement aromatique monocyclique ou un groupement bicyclique dans lequel au moins un des cycles est aromatique, éventuellement substitué par un ou plusieurs groupements, identiques ou différents, halogène, alkyle (C₁-C₆) linéaire ou ramifié, alkoxy (C₁-C₆) linéaire ou ramifié, perhalogénoalkyle (C₁-C₆) linéaire ou ramifié, perhalogénoalkoxy (C₁-C₆) linéaire ou ramifié, hydroxy, cyano, nitro, amino (éventuellement substitué par un ou plusieurs groupements alkyle (C₁-C₆) linéaire ou ramifié), aminosulfonyle (éventuellement substitué par un ou plusieurs groupements alkyle (C₁-C₆) linéaire ou ramifié) ou phényle (éventuellement substitué par un ou plusieurs groupements, identiques ou différents, halogène, alkyle (C₁-C₆) linéaire ou ramifié, perhalogénoalkyle (C₁-C₆) linéaire ou ramifié, hydroxy ou alkoxy (C₁-C₆) linéaire ou ramifié),
◆ par groupement hétéroaryle, on comprend groupement aromatique monocyclique ou un groupement bicyclique dans lequel au moins un des cycles est aromatique, contenant un, deux ou trois hétéroatomes, identiques ou différents, choisis parmi azote, oxygène et soufre, éventuellement substitué par un ou plusieurs groupements, identiques ou différents, halogène, alkyle (C₁-C₆) linéaire ou ramifié, alkoxy (C₁-C₆) linéaire ou ramifié, perhalogénoalkyle (C₁-C₆) linéaire ou ramifié, perhalogénoalkoxy (C₁-C₆) linéaire ou ramifié, hydroxy, cyano, nitro, amino (éventuellement substitué par un ou plusieurs groupements alkyle (C₁-C₆) linéaire ou ramifié) ou aminosulfonyle (éventuellement substitué par un ou plusieurs groupements alkyle (C₁-C₆) linéaire ou ramifié).

Parmi les acides pharmaceutiquement acceptables, on peut citer à titre non limitatif les acides chlorhydrique, bromhydrique, sulfurique, phosphonique, acétique, trifluoroacétique, lactique, pyruvique, malonique, succinique, glutarique, fumarique, tartrique, maléïque, citrique, ascorbique, méthane sulfonique, camphorique, etc...

Parmi les bases pharmaceutiquement acceptables, on peut citer à titre non limitatif l'hydroxyde de sodium, l'hydroxyde de potassium, la triéthylamine, la tertbutylamine, etc... Les groupements aryles préférés sont les groupements phényle, naphtyle et tétrahydronaphtyle éventuellement substitués.

Les groupements hétéroaryles préférés sont les groupements pyridinyle, pyrrolyle, thiényle, furyle, imidazolyle, indolyle éventuellement substitués et de manière encore plus préférentielle les groupements pyridinyle, thiényle et furyle.

Le groupement R₁ est préférentiellement situé en position 7 du composé de formule (I).

Le groupement R₂ est préférentiellement un atome d'hydrogène, les groupements R préférés sont les groupements aryle ou hétéroaryle.

L'invention s'étend également au procédé de préparation des composés de formule (I).

Le procédé de préparation des composés de formule (I) dans laquelle A représente un groupement CR₄R₅ est caractérisé en ce que l'on utilise comme produit de départ un composé de formule (II) : 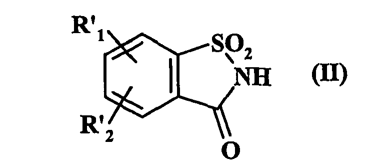 dans laquelle :
- R'₁: représente un groupement alkoxy (C₁-C₆) linéaire ou ramifié,
- R'₂: représente un atome d'hydrogène, d'halogène ou un groupement alkoxy (C₁-C₆) linéaire ou ramifié,
qui subit l'action de la chloroacétone en présence d'une base minérale en milieu diméthylformamide, pour conduire au composé de formule (III) : 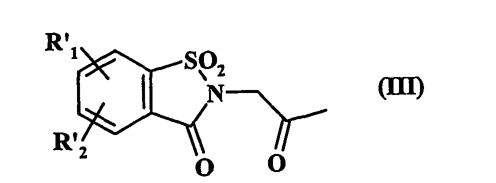 dans laquelle R'₁ et R'₂ ont la même signification que précédemment,
qui subit un réarrangement en milieu basique, pour conduire au composé de formule (IV) : 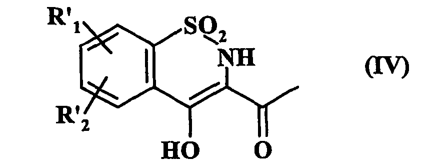 dans laquelle R'₁ et R'₂ ont la même signification que précédemment,
qui est déacétylé par chauffage au reflux en milieu benzénique en présence d'un excès d'éthylèneglycol et d'une quantité catalytique d'acide *p*-toluène sulfonique, pour conduire au composé de formule (V) : 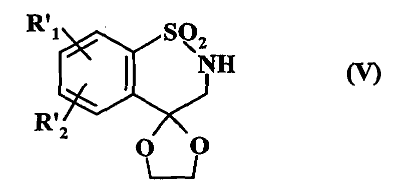 dans laquelle R'₁ et R'₂ ont la même signification que précédemment,
qui subit une hydrolyse en milieu acide, pour conduire au composé de formule (VIa) : 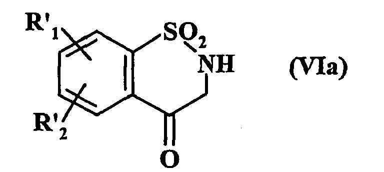 dans laquelle R'₁ et R'₂ ont la même signification que précédemment,
dont, éventuellement, dans le cas particulier où R₃ est différent de l'atome d'hydrogène, on protège l'atome d'azote par un groupement protecteur, puis, après traitement par une base forte, que l'on traite par un composé de formule R'₃ - P,
dans laquelle R'₃ représente un groupement alkyle (C₁-C₆) linéaire ou ramifié ou un groupement cycloalkyle (C₃-C₇) et P représente un groupe partant,
pour conduire après déprotection de l'atome d'azote, au composé de formule (VI'a) : 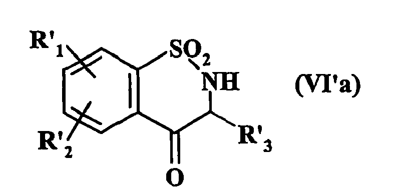 dans laquelle R'₁, R'₂ et R'₃ ont la même signification que précédemment,
composé de formule (VIa) et (VI'a) représentés par la formule (VI) : 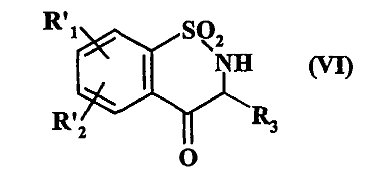 dans laquelle R'₁ et R'₂ ont la même signification et R₃ est tel que défini dans la formule (I),
qui :
- **soit** subit une réduction catalytique, pour conduire au composé de formule (VII) : 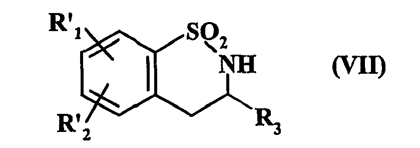 dans laquelle R'₁ et R'₂ ont la même signification que précédemment,
- **soit** est transformé en alcool par l'action d'une hydrure dont on transforme le groupement hydroxy en atome d'halogène par l'action d'un réactif approprié,
   pour conduire au composé de formule (VIII), 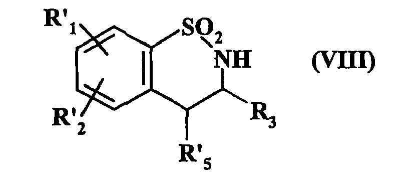 dans laquelle R'₁ et R'₂ ont la même signification que précédemment, R'₅ représente un atome d'halogène
- **soit** subit l'action d'un organomagnésien R'₄ MgBr dans lequel R'₄ représente un groupement alkyle (C₁-C₆) linéaire ou ramifié,
   pour conduire au composé de formule (VIb) : 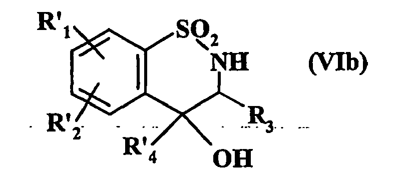 dans laquelle R'₁, R'₂ et R'₄ ont la même signification que précédemment,
   composé de formule (VIb) :
- **ou bien** qui subit une réduction catalytique, pour conduire au composé de formule (IX): 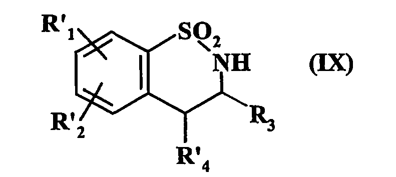 dans laquelle R'₁, R'₂ et R'₄ ont la même signification que précédemment,
- **ou bien** dont on transforme le groupement hydroxy en atome d'halogène par l'action d'un réactif approprié,
   pour conduire au composé de formule (X) : 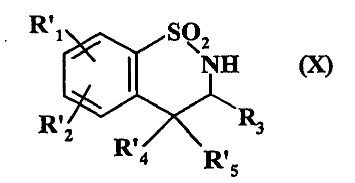
dans laquelle R'₁, R'₂ et R'₄ ont la même signification que précédemment et R'₅ représente un atome d'halogène,
composé de formule (VII) à (X) dont on transforme le groupement R'₁ et le groupement R'₂ lorsque celui-ci représente un groupement alkoxy (C₁-C₆) linéaire ou ramifié, en groupements hydroxy,
pour conduire au composé de formule (I/a), cas particulier des composés de formule (I) : 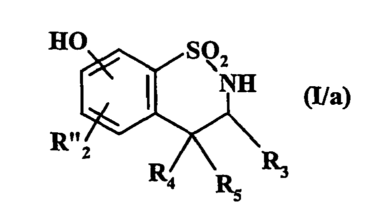 dans laquelle R₄ et R₅ ont la même signification que dans la formule (I) et R"₂ représente un atome d'hydrogène, d'halogène ou un groupement hydroxy,
composé de formule (I/a) dont on transforme, si on le souhaite, la ou les fonctions hydroxy en esters correspondants,
composé de formule (I/a) et ses esters correspondants, qui constituent l'ensemble des composés de formule (I),
que l'on purifie le cas échéant selon une technique classique de purification, dont on sépare éventuellement les isomères selon une technique classique de séparation et que l'on transforme, si on le souhaite, en leurs sels d'addition à un acide ou à une base pharmaceutiquement acceptable.

Le procédé de préparation des composés de formule (I) dans laquelle A représente un groupement NR₄ est caractérisé en ce que l'on utilise comme produit de départ un composé de formule (XI) : 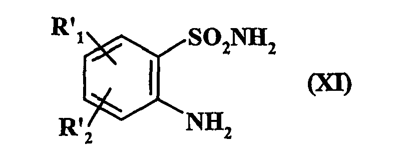 dans laquelle :
R'₁ représente un groupement alkoxy (C₁-C₆) linéaire ou ramifié,
R'₂ représente un atome d'hydrogène, et d'halogène ou un groupement alkoxy (C₁-C₆) linéaire ou ramifié,
qui est cyclisé :
- **soit** en présence d'une amidine de formule (XII): 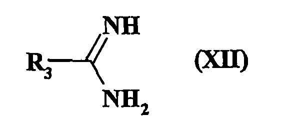 dans laquelle :
   R₃ est tel un défini dans la formule (I),
   pour conduire au composé de formule (XIII): 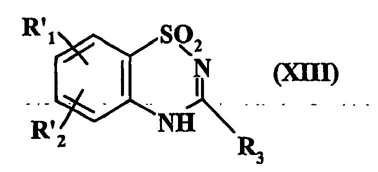 dans laquelle R'₁, R'₂, et R'₃ ont la même signification que précédemment,
   qui est:
   ◆ ou bien réduit par un hydrure métallique,
      pour conduire au composé de formule (XIV) : 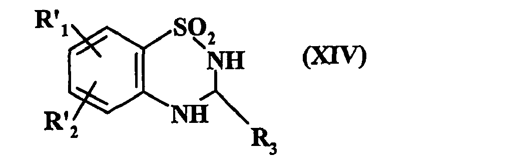 dans laquelle R'₁, R'₂, et R'₃ ont la même signification que précédemment,
   ◆ ou bien alkylé par action d'une base forte en présence d'un agent d'alkylation R'₄X
      dans lequel R'₄ représente un groupement alkyle (C₁-C₆) linéaire ou ramifié et X un atome d'halogène, puis réduit,
      pour conduire au composé de formule (XV) : 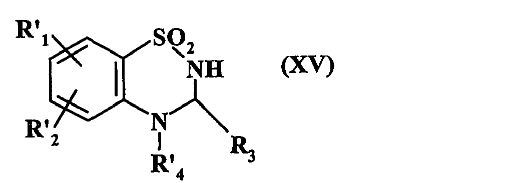
   dans laquelle R'₁, R'₂, R₃ et R'₄ ont la même signification que précédemment,
   - **soit** cyclisé en présence d'un aldéhyde de formule (XVI) : 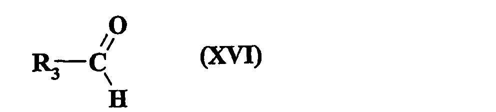
   dans laquelle R₃ est tel que défini dans la formule (I),
   pour conduire au composé de formule (XIV) décrit précédemment,
   composé de formule (XIV) au (XV),
   dont on transforme, le groupement R'₁ et le groupement R'₂ lorsque celui-ci représente un groupement alkoxy (C₁-C₆) linéaire ou ramifié, en groupements hydroxy,
   pour conduire au composé de formule (I/b), cas particulier des composés de formule (I) : 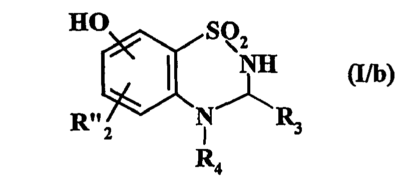 dans laquelle R₃ et R₄ ont la même signification que dans la formule (I) et R"₂ représente un atome d'hydrogène, d'halogène ou un groupement hydroxy,
   composé de formule (I/b) dont on transforme, si on le souhaite, la ou les fonctions hydroxy en esters correspondants,
   composé de formule (I/b) et ses esters correspondants, qui constituent l'ensemble des composés de formule (I),
   que l'on purifie le cas échéant selon une technique classique de purification, dont on sépare éventuellement les isomères selon une technique classique de séparation et que l'on transforme, si on le souhaite, en leurs sels d'addition à un acide ou à une base pharmaceutiquement acceptable.

L'invention s'étend aussi aux compositions pharmaceutiques renfermant comme principe actif un composé de formule (I) avec un ou plusieurs excipients inertes, non toxiques et appropriés. Parmi les compositions pharmaceutiques selon l'invention, on pourra citer plus particulièrement celles qui conviennent pour l'administration orale, parentérale (intraveineuse ou sous-cutanée), nasale, les comprimés simples ou dragéifiés, les comprimés sublinguaux, les gélules, les tablettes, les suppositoires, les crèmes, les pommades, les gels dermiques, les préparations injectables, les suspensions buvables, etc...

La posologie utile est adaptable selon la nature et la sévérité de l'affection, la voie d'administration ainsi que l'âge et le poids du patient. Cette posologie varie de 1 à 5100 mg par jour en une ou plusieurs prises.

Les exemples suivants illustrent l'invention mais ne la limitent en aucune façon.

Les produits de départ utilisés sont des produits connus ou préparés selon des modes opératoires connus.

Les structures des composés décrits dans les exemples ont été déterminées selon les techniques spectrophotométriques usuelles (infrarouge, RMN, spectrométrie de masse ...).

### EXEMPLE 1 : 3,4-Dihydro-2H-1,2-benzathiazin-7-ol 1,1-dioxide

### Stade A : 6-Méthoxy-2-(2-oxopropyl)-1,2-benzisothiazol-3(2H)-one 1,1-dioxide

A une suspension de 72 mg de NaH 60% dans huile minérale dans 1,6 ml de diméthylformamide anhydre, on additionne par petites portions 360 mg de 6-methoxy-1,1-dioxo-1,2-dihydro-benzo[d]isothiazol-3-one. Après 30 min d'agitation à température ambiante, le milieu réactionnel devient homogène et on y ajoute 162 µl de chloroacétone. La réaction est portée à 110°C pendant 30 min. On laisse revenir à température ambiante, puis précipite le milieu par addition d'eau. Le précipité est filtré, rincé plusieurs fois avec de l'eau, essoré et séché sous vide.
***Point de fusion :* 185-191°C**

### Stade B : 2-Acétyl-7-méthoxy-2H-1,2-benzothiazin-4-ol 1,1-dioxide

Une solution d'éthanolate de sodium dans l'éthanol est préparée par dissolution à reflux de 1,08 g de sodium dans 23 ml d'éthanol. On ramène la température de la solution à 40°C et on y ajoute, sous agitation, 6,30 g du produit du stade A. Le milieu réactionnel prend en masse. On ajoute 5 ml d'éthanol pour permettre à l'agitation de se faire et on chauffe 10 min supplémentaires à 50-55°C. Le milieu réactionnel est ensuite refroidi dans un bain de glace. On acidifie avec HCl 3N et on filtre le précipité jaune qui se forme.
***Point de fusion :* 162-166°C**

### Stade C: 7-Méthoxy-2,3-dihydro-4H-1,2-benzothiazine-4,4-éthylènedioxy 1,1-dioxide

On agite au reflux dans un ballon surmonté d'un Dean-Stark 5,35 g du produit obtenu au stade précédent, 200 mg d'acide paratoluène sulfonique, 5,6 ml d'éthylène glycol dans 200 ml de benzène. Après 72 h de reflux le benzène est évaporé sous vide. Le résidu est dissous dans de l'acétate d'éthyle et la phase organique est lavée avec de l'eau puis NaCl saturé. On sèche, filtre, évapore et on obtient une huile que l'on cristallise dans un mélange éther éthylique / éther isopropylique.
***Point de fusion :* 100-110 °C**

### Stade D: 7-Méthoxy-2,3-dihydro-4H-1,2-benzothiazin-4-one 1,1-dioxide

On agite au reflux pendant 15 min une solution de 2,63 g du produit du stade précédent dans un mélange de 50 ml de méthanol et 50 ml d'HCl 3 N. Le méthanol est évaporé sous vide et on extrait la phase aqueuse avec de l'éther. La phase organique est séchée et traitée au noir animal. Après filtration et évaporation le résidu est repris dans de l'éther isopropylique et on filtre le solide.
***Point de fusion* 124-127°C**

### Stade E : 7-Méthoxy-3,4-dihydro-2H-1,2-benzothiazine 1,1-dioxide

On hydrogène sous 5 bars à 70 °C 1,77 g du produit du stade précédent dans 40 ml d'acide acétique en présence de 1.75 g de Pd/C 10%. On laisse revenir à température ambiante et filtre le catalyseur. Le filtrat est évaporé à sec et le résidu est chromatographié sur silice en éluant avec un système 95/5 chlorure de méthylène/acétate d'éthyle pour conduire au produit attendu.
***Point de fusion :* 144-145°C**

### Stade F: 3,4-Dihydro-2H-1,2-benzothiazin-7-ol 1,1-dioxide

A une solution de 1 g du produit du stade précédent dans 45 ml de chlorure de méthylène refroidit à -35 °C on ajoute goutte à goutte 14,1 ml d'une solution 1M de BBr₃ dans le chlorure de méthylène. On laisse revenir à température ambiante. Après 3h d'agitation à température ambiante, le milieu réactionnel est versé dans de l'eau à 5°C et on extrait à l'acétate d'éthyle. On joint les phases organiques, lave avec NaCl saturé, sèche, filtre, évapore. On obtient un solide qui est repris dans un peu d'éther isopropylique. On filtre le produit titre.
**P*oint de fusion :* 173-177 °C**

| ***Microanalyse élémentaire :*** | | | | |
|---|---|---|---|---|
| | C | *H* | *N* | *S* |
| *% théorique* | *48,23* | *4,55* | *7, 03* | *16, 09* |
| *% expérimental* | *48,53* | *4,54* | *7,16* | *15,82* |

### EXEMPLE 2 : 1,1-Dioxido-3,4-dihydra-2H-1,2-benzothiazin-7-yl benzoate

Le produit attendu est obtenu par benzoylation du composé de l'exemple 1 avec le chlorure de l'acide benzoique dans le dichlorométhane en présence de 1,5 eq. de triéthylamine et une quantité catalytique de diméthylaminopyridine.
***Point de fusion* : 153-156 °C**

| ***Microanalyse élémentaire :*** | | | | |
|---|---|---|---|---|
| | **C** | ***H*** | ***N*** | ***S*** |
| ***% théorique*** | ***59,39*** | ***4,32*** | ***4,62*** | ***10,57*** |
| ***% expérimental*** | ***59,48*** | ***4,38*** | ***4,69*** | ***10,67*** |

### EXEMPLE 3 : 3-Méthyl-1,1-dioxido-3,4-dihydro-2H-1,2,4-benzothiadiazin-7-yl benzoate

### Stade A : 7-Méthoxy-3-méthyl-3,4-dihydro-2H 1,2,4-benzothiadiazine 1,1-dioxide

A une suspension de 20 g de 2-amino-5-methoxy-benzenesulfonamide dans 20 ml d'éthanol anhydre, on ajoute 829 µl d'acétaldéhyde. Le milieu réactionnel devient homogène et on le chauffe au reflux pendant 30 min. On évapore l'éthanol sous vide. Le solide est repris dans de l'éther et on le filtre. On reprend le solide dans 40 ml d'éthanol porte au reflux pendant 15 min et laisse revenir à température ambiante. Après filtration on obtient le produit attendu.
***Point de fusion :* 208-218 °C**

### Stade B : 3-Méthyl-3,4-dihydro-2H-1,2,4-benzothiadiazin-7-ol 1,1-dioxide

A une suspension de 1,70 g du produit du stade précédent dans 85 ml de chlorure de méthylène refroidi à -65 °C, on additionne goutte à goutte 1,76 ml de BBr₃. On agite la réaction 45 min à -65°C puis on laisse la température remonter à l'ambiante. Après 3 h à température ambiante on verse sur de la glace. Lorsque la glace est fondue, on extrait la phase aqueuse avec de l'acétate d'éthyle. On joint les phases organiques, lave et sèche. Le produit attendu est purifié par chromatographie sur silice en éluant avec un système 98/2 de chlorure de méthylène / méthanol.
***Point de fusion :* 210-212 °C**

### Stade C:3-Méthyl-1,1-dioxido-3,4-dihydro-2H-1,2,4-benzothiadiazin-7-yl benzoate

Le produit attendu est obtenu par benzoylation du produit décrit au stade B avec le chlorure de l'acide benzoïque dans le dichlorométhane en présence de 1,5 eq. de triéthylamine et une quantité catalytique de diméthylaminopyridine.
***Point de fusion* : 209-211 °C**

| ***Microanalyse élémentaire :*** | | | | |
|---|---|---|---|---|
| | ***C*** | ***H*** | ***N*** | ***S*** |
| ***% théorique*** | ***56,59*** | ***4,43*** | ***8,80*** | ***10,07*** |
| ***% expérimental*** | ***56,56*** | ***4,43*** | ***8,68*** | ***10,25*** |

### EXEMPLE 4: 4-Méthyl-3,4-dihydro-2H-1,2,4-benzothiadiazin-7-ol 1,1-dioxide

### Stade A : 7-Méthoxy-4H-1,2,4-benzothiadiazine 1,1-dioxide

On agite 1 nuit à 80°C une suspension de 3,0 g de 2-amino-5-methoxy-benzenesulfonamide en présence de 1,31 g de chlorhydrate de formamidine et de 2,27 ml de triéthylamine dans 50 ml de toluène. Le toluène est évaporé sous vide. Le résidu est repris dans de l'eau et on filtre le précipité.

### Point de fusion : 253-257°C

### Stade B : 7-Méthoxy-4-méthyl-4H-1,2,4-benzothiadiazine 1,1-dioxide

A une suspension de 9 ml de DMF contenant 570 mg du NaH 60 % dans huile minérale, on additionne portion par portion 2,88 g du produit obtenu au stade précédent. On agite 30 min jusqu'à l'obtention d'une solution noire. A celle-ci on ajoute alors goutte à goutte 929 µl d'iodométhane. On poursuit l'agitation 1 h et on précipite le milieu réactionnel par addition d'eau. Le précipité est filtré, rincé à l'eau puis à l'éther pour donner le produit attendu.
***Point de fusion :* 205-209°C**

### Stade C : 7-Méthoxy-4-méthyl-3,4-dihydro-2H-1,2,4-benzothiadiazine 1,1-dioxide

A une suspension de 2,37 g du produit du stade précédent dans 40 ml d'éthanol, on ajoute 1,19 g de borohydrure de sodium. Le milieu devient peu à peu homogène. Après 1 h de réaction à température ambiante on refroidit dans un bain de glace et neutralise par addition de HCl 1N. On agite le précipité blanc pendant 15 min et on filtre le produit du titre.
***Point de fusion:* 126-128°C**

### Stade D : 4-Méthyl-3,4-dihydro-2H-1,2,4-benzothiadiazin-7-ol 1,1-dioxide

A une suspension contenant 2 g du produit obtenu au stade précédent dans 200 ml de dichlorométhane maintenue à -60°C sous azote sont ajoutés goutte à goutte 79,3 mmoles de tribromure de bore. La température est maintenue une heure puis l'ensemble revient à température ambiante et est agité une nuit. Après refroidissement du milieu réactionnel dans un bain de glace, 100 ml d'eau sont ajoutés et le système biphasique est agité vigoureusement. La suspension ainsi formée est filtrée. Le solide obtenu est lavé à l'eau, à l'éther, séché et conduit au produit attendu.
***Point de fusion:* 168-172°C**

| ***Microanalyse élémentaire :*** | | | | |
|---|---|---|---|---|
| | ***C*** | ***H*** | ***N*** | ***S*** |
| ***% théorique*** | ***44.85*** | ***4.70*** | ***13.08*** | ***14.97*** |
| ***% expérimental*** | ***45.10*** | ***4.83*** | ***12.64*** | ***14.77*** |

### EXEMPLE 5 : 4-Méthyl-1,1-dioxido-3,4-dihydro-2H-1,2,4-benzothiadiazin-7-yl benzoate

Le produit attendu est obtenu par benzoylation du composé de l'exemple 4 avec le chlorure de l'acide benzoique dans le dichlorométhane en présence de 1,5 eq. de triéthylamine et une quantité catalytique de diméthylaminopyridine.
***Point de fusion* : 199-201 °C**

| ***Microanalyse élémentaire :*** | | | | |
|---|---|---|---|---|
| | ***C*** | ***H*** | ***N*** | ***S*** |
| ***% théorique*** | ***56,59*** | ***4,43*** | ***8,80*** | ***10,07*** |
| ***% expérimental*** | ***56,71*** | ***4,37*** | ***8,56*** | ***10,03*** |

### EXEMPLE 6 :4-Ethyl-3,4-dihydro-2H-1,2,4-benzothiadiazin-7-ol 1,1-dioxide

Le produit attendu est obtenu selon le procédé décrit dans l'exemple 4 en remplaçant au stade B l'iodométhane par l'iodoéthane.
***Point de fusion :* 214-218 °C**

| ***Microanalyse élémentaire :*** | | | | |
|---|---|---|---|---|
| | ***C*** | ***H*** | ***N*** | ***S*** |
| ***% théorique*** | ***47,36*** | ***5,30*** | ***12,27*** | ***14,05*** |
| ***% expérimental*** | ***47,07*** | ***5,52*** | ***11,90*** | ***14,00*** |

### EXEMPLE 7 : 4-Ethyl-1,1-dioxido-3,4-dihydro-2H-1,2,4-benzothiadiazin-7-yl benzoate

Le produit attendu est obtenu par benzoylation du composé de l'exemple 6 avec le chlorure de l'acide benzoique dans le dichlorométhane en présence de 1,5 eq. de triéthylamine et une quantité catalytique de diméthylaminopyridine.
***Point de fusion : 148-152 °C***

| ***Microanalyse élémentaire :*** | | | | |
|---|---|---|---|---|
| | ***C*** | ***H*** | ***N*** | ***S*** |
| ***% théorique*** | ***57,82*** | ***4,85*** | ***8,43*** | ***9,65*** |
| ***% expérimental*** | ***57,87*** | ***4,94*** | ***8,21*** | ***9,67*** |

### EXEMPLE 8 : 3-Cyclohexyl-3,4-dihydra-2H-1,2,4-benzothiadiazin-7-ol 1,1-dioxide

Le produit est obtenu selon le procédé décrit dans l'exemple 3 en remplaçant au stade A l'acétaldéhyde par le cyclohexylcarboxaldéhyde.
***Point de fusion* : 268-273 °C**

| ***Microanalyse élémentaire :*** | | | | |
|---|---|---|---|---|
| | ***C*** | ***H*** | ***N*** | ***S*** |
| ***% théorique*** | ***55,30*** | ***6,43*** | ***9,92*** | ***11,36*** |
| ***% expérimental*** | ***54,81*** | ***6,38*** | ***9,44*** | ***11,12*** |

### EXEMPLE 9: 3-Cyclohexyl-1,1-clioxido-3,4-dihydro-2H-1,2,4-benzothiadiazin-7-yl benzoate

Le produit attendu est obtenu par benzoylation du composé de l'exemple 8 avec le chlorure de l'acide benzoique dans le dichlorométhane en présence de 1,5 eq. de triéthylamine et une quantité catalytique de diméthylaminopyridine.
***Point de fusion : 214-218 °C***

| ***Microanalyse élémentaire :*** | | | | |
|---|---|---|---|---|
| | **C** | ***H*** | ***N*** | ***S*** |
| ***% théorique*** | ***62,16*** | ***5,74*** | ***7,25*** | ***8,3*** |
| ***% expérimental*** | ***61,86*** | ***5,84*** | ***7,05*** | ***8,27*** |

### ETUDE PHARMACOLOGIQUE DES PRODUITS DE L'INVENTION

### Etude des courants excitateurs induit par l'AMPA dans les oocytes de Xenopus

### a - Méthode :

Des ARNm sont préparés à partir de cortex cérébral de rat mâle Wistar par la méthode au guanidium thiocyanate/phénol/chloroforme. Les ARNm Poly (A⁺) sont isolés par chromatographie sur oligo-dT cellulose et injectés à raison de 50 ng par oocyte. Les oocytes sont laissés 2 à 3 jours en incubation à 18°C pour permettre l'expression des récepteurs puis stockés à 8-10°C.

L'enregistrement électrophysiologique est réalisé dans une chambre en plexiglass^{®} à 20-24°C en milieu OR2 (J. Exp. Zool., 1973, 184, 321-334) par la méthode du "voltage-clamp" à 2 électrodes, une 3ème électrode placée dans le bain servant de référence.

Tous les composés sont appliqués via le milieu d'incubation et le courant électrique est mesuré à la fin de la période d'application. L'AMPA est utilisé à la concentration de 10 µM. Pour chaque composé étudié, on définit la concentration doublant (EC2X) ou quintuplant (EC5X) l'intensité du courant induit par l'AMPA seul (5 à 50 nA).

### b - Résultats :

Les composés de l'invention potentialisent très fortement les effets excitateurs de l'AMPA et leur activité est très nettement supérieure à celles des composés de référence.

Le composé de l'exemple 2 possède notamment une EC2X égale à 11,9 µM et une EC5X égale à 49,3 µM.

**COMPOSITION PHARMACEUTIQUE**

| Formule de préparation pour 1000 comprimés dosés à 100 mg | |
|---|---|
| Composé de l'exemple 1 | 100 g |
| Hydroxypropylcellulose | 2 g |
| Amidon de blé | 10 g |
| Lactose | 100 g |
| Stéarate de magnésium | 3 g |
| Talc | 3 g |

## Revendications

1. Composés de formule (I) : 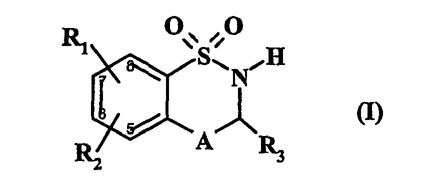 dans laquelle :
**R₁** représente un groupement hydroxy ou un groupement RCO-O-,
**R₂** représente un atome d'hydrogène, d'halogène, un groupement hydroxy ou un groupement R'CO-O,
**R, R',** identiques ou différents, représentent un groupement alkyle (C₁-C₆) linéaire ou ramifié éventuellement substitué par un groupement aryle, alkényle (C₂-C₆) linéaire ou ramifié éventuellement substitué par un groupement aryle, perhalogénoalkyle (C₁-C₆) linéaire ou ramifié, cycloalkyle (C₃-C₇), adamantyle, aryle ou hétéroaryle,
**R₃** représente un atome d'hydrogène, un groupement alkyle (C₁-C₆) linéaire ou ramifié ou un groupement cycloalkyle (C₃-C₇),
**A** représente un groupement CR₄R₅ ou un groupement NR₄,
**R₄** représente un atome d'hydrogène ou un groupement alkyle (C₁-C₆) linéaire ou ramifié,
**R₅** représente un atome d'hydrogène ou d'halogène,
leurs isomères ainsi que leurs sels d'addition à un acide ou à une base pharmaceutiquement acceptable,
étant entendu que:
◆ R₁ représente un groupement RCO-O- lorsqu'il est situé en position 5 du composé de formule (I),
◆ par groupement aryle, on comprend groupement aromatique monocyclique ou un groupement bicyclique dans lequel au moins un des cycles est aromatique, éventuellement substitué par un ou plusieurs groupements, identiques ou différents, halogène, alkyle (C₁-C₆) linéaire ou ramifié, alkoxy (C₁-C₆) linéaire ou ramifié, perhalogénoalkyle (C₁-C₆) linéaire ou ramifié, perhalogénoalkoxy (C₁-C₆) linéaire ou ramifié, hydroxy, cyano, nitro, amino (éventuellement substitué par un ou plusieurs groupements alkyle (C₁-C₆) linéaire ou ramifié), aminosulfonyle (éventuellement substitué par un ou plusieurs groupements alkyle (C₁-C₆) linéaire ou ramifié) ou phényle (éventuellement substitué par un ou plusieurs groupements, identiques ou différents, halogène, alkyle (C₁-C₆) linéaire ou ramifié, perhalogénoalkyle (C₁-C₆) linéaire ou ramifié, hydroxy ou alkoxy (C₁-C₆) linéaire ou ramifié),
◆ par groupement hétéroaryle, on comprend groupement aromatique monocyclique ou un groupement bicyclique dans lequel au moins un des cycles est aromatique, contenant un, deux ou trois hétéroatomes, identiques ou différents, choisis parmi azote, oxygène et soufre, éventuellement substitué par un ou plusieurs groupements, identiques ou différents, halogène, alkyle (C₁-C₆) linéaire ou ramifié, alkoxy (C₁-C₆) linéaire ou ramifié, perhalogénoalkyle (C₁-C₆) linéaire ou ramifié, perhalogénoalkoxy (C₁-C₆) linéaire ou ramifié, hydroxy, cyano, nitro, amino (éventuellement substitué par un ou plusieurs groupements alkyle (C₁-C₆) linéaire ou ramifié) ou aminosulfonyle (éventuellement substitué par un ou plusieurs groupements alkyle (C₁-C₆) linéaire ou ramifié).

2. Composés de formule (I) selon la revendication 1 **caractérisés en ce que** A représente un groupement CR₄R₅.

3. Composés de formule (I) selon la revendication 1 **caractérisés en ce que** A représente NR₄.

4. Composés de formule (I) selon l'une quelconque des revendications 1 à 3 **caractérisés en ce que** le groupement R₁ est situé en position 7 du composé de formule (I).

5. Composés de formule (I) selon l'une quelconque des revendications 1 à 4 **caractérisés en ce que** R₁ représente un groupement hydroxy.

6. Composés de formule (I) selon l'une quelconque des revendications 1 à 4 **caractérisés en ce que** R₁ représente un groupement RCO-O-.

7. Composés de formule (I) selon l'une quelconque des revendications 1 à 6 **caractérisés en ce que** R₂ représente un atome d'hydrogène.

8. Composé de formule (I) selon la revendication 1 qui est le 1,1-dioxido-3,4-dihydro-2*H-*1,2-benzothiazin-7yl benzoate.

9. Procédé de préparation des composés de formule (I) dans laquelle A représente un groupement CR₄R₅ est **caractérisé en ce que** l'on utilise comme produit de départ un composé de formule (II) : 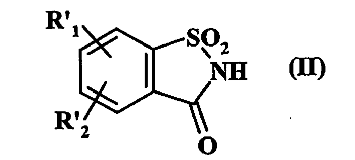 dans laquelle :
R'₁ représente un groupement alkoxy (C₁-C₆) linéaire ou ramifié,
R'₂ représente un atome d'hydrogène, d'halogène ou un groupement alkoxy (C₁-C₆) linéaire ou ramifié,
qui subit l'action de la chloroacétone en présence d'une base minérale en milieu diméthylformamide, pour conduire au composé de formule (III): 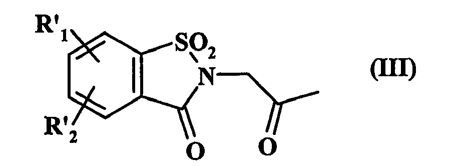 dans laquelle R'₁ et R'₂ ont la même signification que précédemment,
qui subit un réarrangement en milieu basique, pour conduire au composé de formule (IV) : 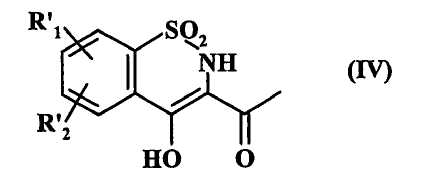 dans laquelle R'₁ et R'₂ ont la même signification que précédemment,
qui est déacétylé par chauffage au reflux en milieu benzénique en présence d'un excès d'éthylèneglycol et d'une quantité catalytique d'acide *p*-toluène sulfonique, pour conduire au composé de formule (V) : 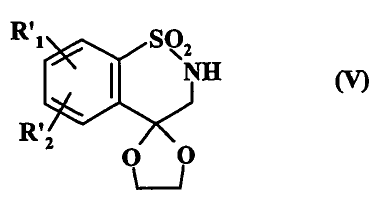 dans laquelle R'₁, et R'₂ ont la même signification que précédemment,
qui subit une hydrolyse en milieu acide, pour conduire au composé de formule (VIa) : 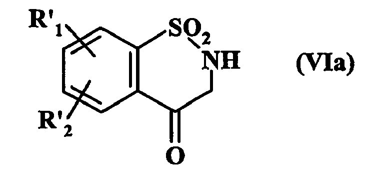 dans laquelle R'₁ et R'₂ ont la même signification que précédemment,
dont, éventuellement, dans le cas particulier où R₃ est différent d'un atome d'hydrogène, on protège l'atome d'azote par un groupement protecteur, puis, après traitement par une base forte, que l'on traite par un composé de formule R'₃ - P,
dans laquelle R'₃ représente un groupement alkyle (C₁-C₆) linéaire ou ramifié ou un groupement cycloalkyle (C₃-C₇) et P représente un groupe partant,
pour conduire après déprotection de l'atome d'azote, au composé de formule (VI'a) : 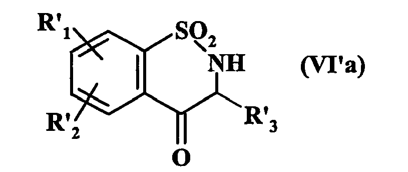 dans laquelle R'₁, R'₂ et R'₃ ont la même signification que précédemment,
composé de formule (VIa) et (VI'a) représentés par la formule (VI) : 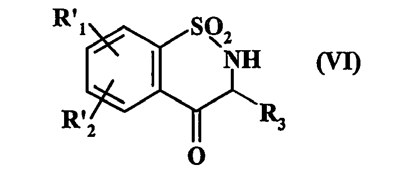 dans laquelle R'₁ et R'₂ ont la même signification et R₃ est tel que défini dans la formule (I),
qui :
- **soit** subit une réduction catalytique, pour conduire au composé de formule (VII) : 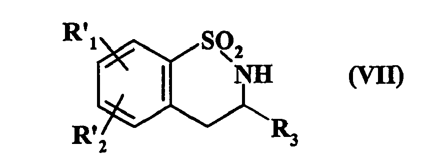 dans laquelle R'₁, et R'₂ ont la même signification que précédemment,
- **soit** est transformé en alcool par l'action d'une hydrure dont on transforme le groupement hydroxy en atome d'halogène par l'action d'un réactif approprié,
pour conduire au composé de formule (VIII) : 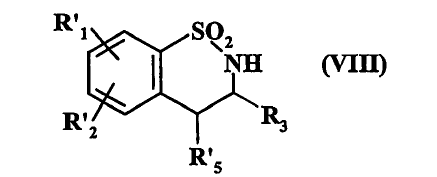 dans laquelle R'₁ et R'₂ ont la même signification que précédemment, R'₅ représente un atome d'halogène
- **soit** subit l'action d'un organomagnésien R'₄ MgBr dans lequel R'₄ représente un groupement alkyle (C₁-C₆) linéaire ou ramifié,
pour conduire au composé de formule (VIb) : 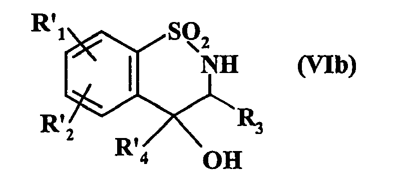 dans laquelle R'₁, R'₂ et R'₄ ont la même signification que précédemment,
composé de formule (VIb) :
- **ou bien** qui subit une réduction catalytique, pour conduire au composé de formule (IX) : 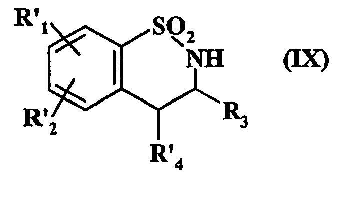 dans laquelle R'₁, R'₂ et R'₄ ont la même signification que précédemment,
- **ou bien** dont on transforme le groupement hydroxy en atome d'halogène par l'action d'un réactif approprié,
pour conduire au composé de formule (X) : 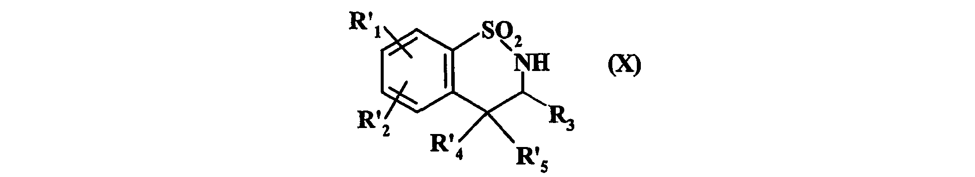
dans laquelle R'₁, R'₂ et R'₄ ont la même signification que précédemment et R'₅ représente un atome d'halogène,
composé de formule (VII) à (X) dont on transforme le groupement R'₁ et le groupement R'₂ lorsque celui-ci représente un groupement alkoxy (C₁-C₆) linéaire ou ramifié, en groupements hydroxy,
pour conduire au composé de formule (I/a), cas particulier des composés de formule (I) : 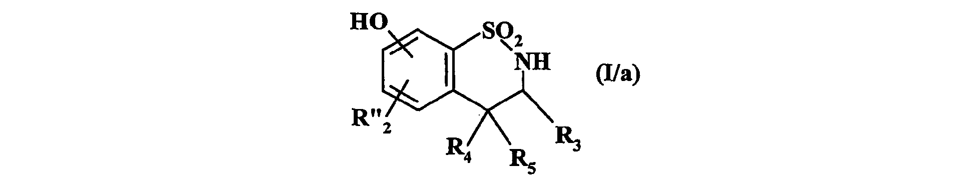 dans laquelle R₄ et R₅ ont la même signification que dans la formule (I) et R"₂ représente un atome d'hydrogène, d'halogène ou un groupement hydroxy,
composé de formule (I/a) dont on transforme, si on le souhaite, la ou les fonctions hydroxy en esters correspondants,
composé de formule (I/a) et ses esters correspondants, qui constituent l'ensemble des composés de formule (I),
que l'on purifie le cas échéant selon une technique classique de purification, dont on sépare éventuellement les isomères selon une technique classique de séparation et que l'on transforme, si on le souhaite, en leurs sels d'addition à un acide ou à une base pharmaceutiquement acceptable.

10. Procédé de préparation des composés de formule (I) dans laquelle A représente un groupement NR₄ est **caractérisé en ce que** l'on utilise comme produit de départ un composé de formule (XI) : 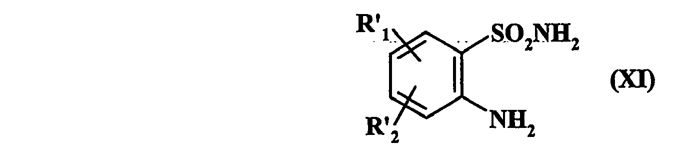 dans laquelle :
R'₁ représente un groupement alkoxy (C₁-C₆) linéaire ou ramifié,
R'₂ représente un atome d'hydrogène, et d'halogène ou un groupement alkoxy (C₁-C₆) linéaire ou ramifié,
qui est cyclisé :
- **soit** en présence d'une amidine de formule (XII) : 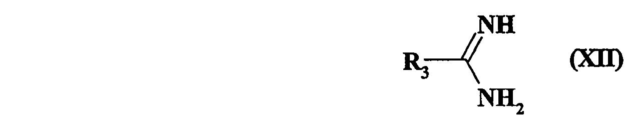 dans laquelle :
R₃ est tel un défini dans la formule (I),
pour conduire au composé de formule (XIII) : 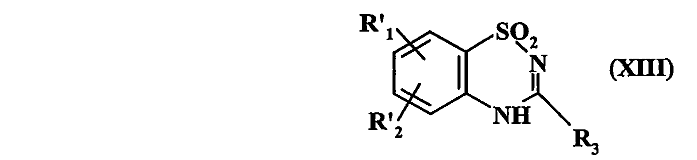 dans laquelle R'₁, R'₂, et R'₃ ont la même signification que précédemment,
qui est :
◆ ou bien réduit par un hydrure métallique,
pour conduire au composé de formule (XIV) : 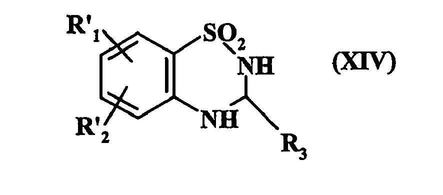 dans laquelle R'₁, R'₂, et R'₃ ont la même signification que précédemment,
◆ ou bien alkylé par action d'une base forte en présence d'un agent d'alkylation R'₄X dans lequel R'₄ représente un groupement alkyle (C₁-C₆) linéaire ou ramifié et X un atome d'halogène, puis réduit,
pour conduire au composé de formule (XV) : 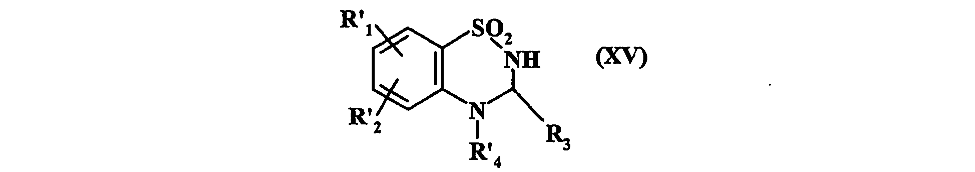 dans laquelle R'₁, R'₂, R₃ et R'₄ ont la même signification que précédemment,
- **soit** cyclisé en présence d'un aldéhyde de formule (XVI) 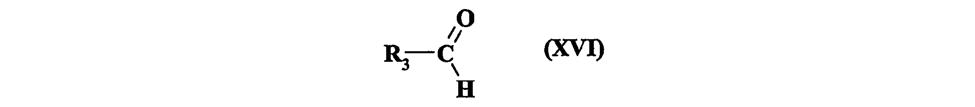
dans laquelle R₃ est tel que défini dans la formule (I),
pour conduire au composé de formule (XIV) décrit précédemment,
composé de formule (XIV) au (XV)
dont on transforme, le groupement R'₁ et le groupement R'₂ lorsque celui-ci représente un groupement alkoxy (C₁-C₆) linéaire ou ramifié, en groupements hydroxy,
pour conduire au composé de formule (I/b), cas particulier des composés de formule (I) : 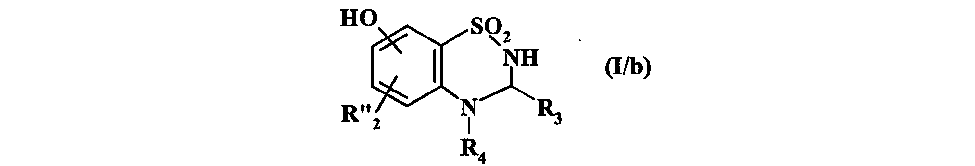 dans laquelle R₃ et R₄ ont la même signification que dans la formule (I) et R"₂ représente un atome d'hydrogène, d'halogène ou un groupement hydroxy,
composé de formule (I/b) dont on transforme, si on le souhaite, la ou les fonctions hydroxy en esters correspondants,
composé de formule (I/b) et ses esters correspondants, qui constituent l'ensemble des composés de formule (I),
que l'on purifie le cas échéant selon une technique classique de purification, dont on sépare éventuellement les isomères selon une technique classique de séparation et que l'on transforme, si on le souhaite, en leurs sels d'addition à un acide ou à une base pharmaceutiquement acceptable.

11. Compositions pharmaceutiques contenant comme principe actif un composé selon l'une quelconque des revendications 1 à 8 en combinaison avec un ou plusieurs excipients ou véhicules inertes, non toxiques, pharmaceutiquement acceptables.

12. Compositions pharmaceutiques selon la revendication 11 contenant comme principe actif un composé selon l'une quelconque des revendications 1 à 8 utiles en tant que médicaments, comme modulateurs AMPA.

## Claims

1. Compounds of formula (I) : 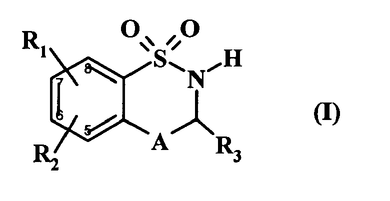 wherein:
**R₁** represents a hydroxy group or a RCO-O- group,
**R₂** represents a hydrogen atom, a halogen atom, a hydroxy group or a R'CO-O- group,
**R, R',** which may be identical or different, represent a linear or branched (C₁-C₆)alkyl group optionally substituted by an aryl group, a linear or branched (C₂-C₆)alkenyl group optionally substituted by an aryl group, a linear or branched (C₁-C₆)perhaloalkyl group, a (C₃-C₇)cycloalkyl group, an adamantyl group, an aryl group or a heteroaryl group,
**R₃** represents a hydrogen atom, a linear or branched (C₁-C₆)alkyl group or a (C₃-C₇)-cycloalkyl group,
**A** represents a CR₄R₅ group or an NR₄ group,
**R₄** represents a hydrogen atom or a linear or branched (C₁-C₆)alkyl group,
**R₅** represents a hydrogen atom or a halogen atom,
their isomers and their addition salts with a pharmaceutically acceptable acid or base, it being understood that :
◆ R₁ represents an RCO-O- group when it is located in the 5 position of the compound of formula (I),
◆ "aryl group" is understood to mean an aromatic monocyclic group, or a bicyclic group in which at least one of the rings is aromatic, optionally substituted by one or more identical or different groups: halogen, linear or branched (C₁-C₆)alkyl, linear or branched (C₁-C₆)alkoxy, linear or branched (C₁-C₆)perhaloalkyl, linear or branched (C₁-C₆)perhaloalkoxy, hydroxy, cyano, nitro, amino (optionally substituted by one or more linear or branched (C₁-C₆)alkyl groups), aminosulphonyl (optionally substituted by one or more linear or branched (C₁-C₆)alkyl groups) or phenyl (optionally substituted by one or more identical or different groups: halogen, linear or branched (C₁-C₆)alkyl, linear or branched (C₁-C₆)perhaloalkyl, hydroxy or linear or branched (C₁-C₆)alkoxy),
◆ "heteroaryl group" is understood to mean an aromatic monocyclic group, or a bicyclic group in which at least one of the rings is aromatic, containing one, two or three identical or different hetero atoms selected from nitrogen, oxygen and sulphur, optionally substituted by one or more identical or different groups: halogen, linear or branched (C₁-C₆)alkyl, linear or branched (C₁-C₆)alkoxy, linear or branched (C₁-C₆)perhaloalkyl, linear or branched (C₁-C₆)perhaloalkoxy, hydroxy, cyano, nitro, amino (optionally substituted by one or more linear or branched (C₁-C₆)alkyl groups) or aminosulphonyl (optionally substituted by one or more linear or branched (C₁-C₆)-alkyl groups).

2. Compounds of formula (I) according to claim 1 **characterised in that** A represents a CR₄R₅ group.

3. Compounds of formula (I) according to claim 1 **characterised in that** A represents NR₄.

4. Compounds of formula (I) according to any one of claims 1 to 3 **characterised in that** the group R₁ is located in the 7 position of the compound of formula (I).

5. Compounds of formula (I) according to any one of claims 1 to 4 **characterised in that** R₁ represents a hydroxy group.

6. Compounds of formula (I) according to any one of claims 1 to 4 **characterised in that** R₁ represents a RCO-O- group.

7. Compounds of formula (I) according to any one of claims 1 to 6 **characterised in that** R₂ represents a hydrogen atom.

8. Compound of formula (I) according to claim 1 which is 1,1-dioxido-3,4-dihydro-2*H-*1,2-benzothiazin-7-yl benzoate.

9. Process for the preparation of compounds of formula (I) wherein A represents a CR₄R₅ group is **characterised in that** there is used as starting material a compound of formula **(II)** : 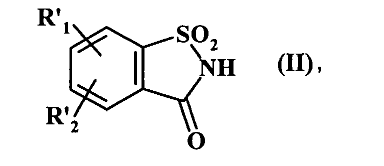 wherein
R'₁ represents a linear or branched (C₁-C₆)alkoxy group,
R'₂ represents a hydrogen atom, a halogen atom or a linear or branched (C₁-C₆)alkoxy group,
which is subjected to the action of chloroacetone in the presence of a mineral base in dimethylformamide medium to yield the compound of formula (III) : 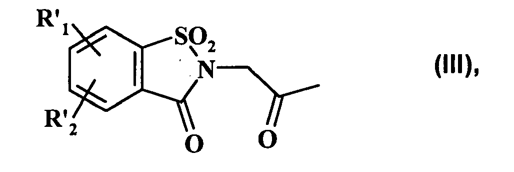 wherein R'₁ and R'₂ are as defined hereinbefore,
which is subjected to rearrangement in basic medium to yield the compound of formula (IV) : 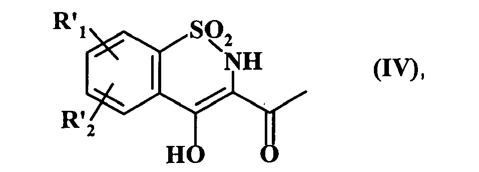 wherein R'₁ and R'₂ are as defined hereinbefore,
which is deacetylated by heating at reflux in benzene medium in the presence of an excess of ethylene glycol and a catalytic amount of *p*-toluenesulphonic acid to yield the compound of formula (V) : 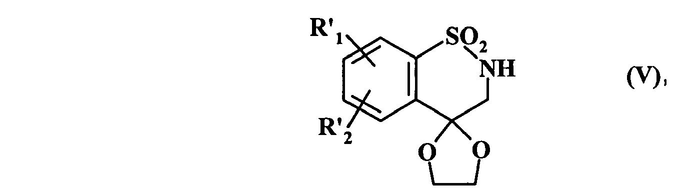 wherein R'₁ and R'₂ are as defined hereinbefore,
which is subjected to hydrolysis in acid medium to yield the compound of formula (VIa) : 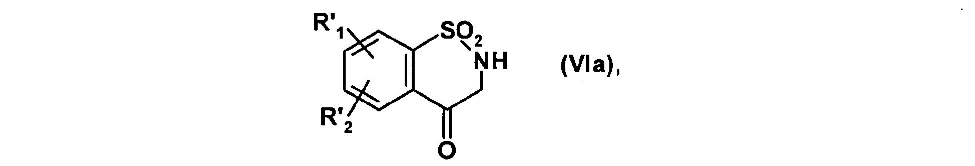 wherein R'₁ and R'₂ are as defined hereinbefore,
of which, optionally, when R₃ is other than a hydrogen atom, the nitrogen atom is protected by a protecting group, and which then, after treatment with a strong base, is treated with a compound of formula R'₃-P,
wherein R'₃ represents a linear or branched (C₁-C₆)alkyl group or a (C₃-C₇)cycloalkyl group and P represents a leaving group,
to yield, after deprotection of the nitrogen atom, the compound of formula (VI'a): 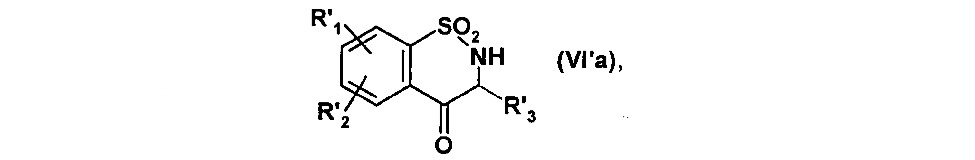 wherein R'₁, R'₂ and R'₃ are as defined hereinbefore,
which compound of formula (VIa) or (VI'a), represented by formula (VI) : 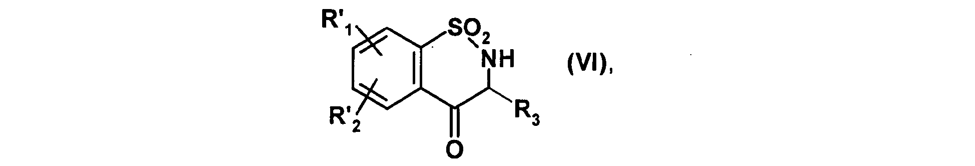 wherein R'₁ and R'₂ have the same meaning and R₃ is as defined for formula (I),
is :
- **either** subjected to catalytic reduction to yield the compound of formula (VII) : 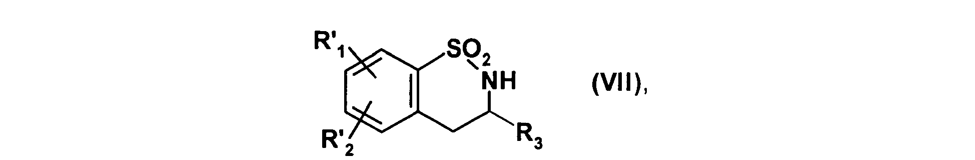 wherein R'₁ and R'₂ are as defined hereinbefore,
- **or** converted in alcohol by the action of a hydride, the hydroxy group of the product then being converted to a halogen atom by the action of an appropriate reagent,
to yield the compound of formula (VIII) : 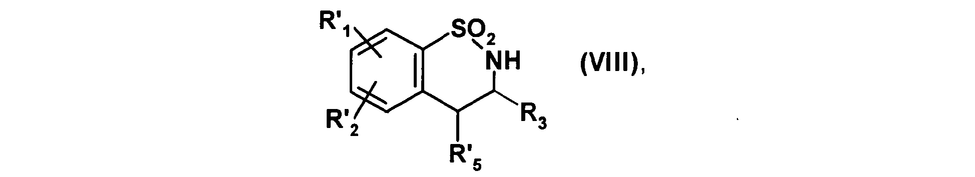 wherein R'₁ and R'₂ are as defined hereinbefore, R'₅ represents a halogen atom,
- **or** subjected to the action of an organomagnesium compound R'₄ MgBr wherein R'₄ represents a linear or branched (C₁-C₆)alkyl group,
to yield the compound of formula (VIb) : 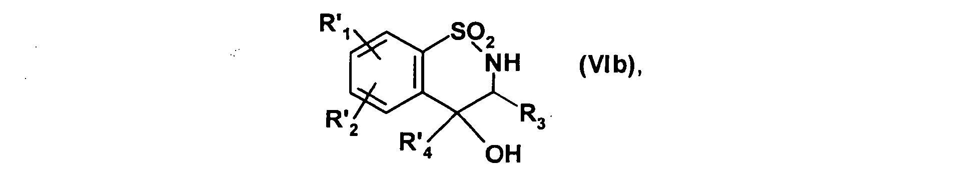 wherein R'₁, R'₂ and R'₄ are as defined hereinbefore,
which compound of formula (VIb) :
- **is either** subjected to catalytic reduction to yield the compound of formula (IX) : 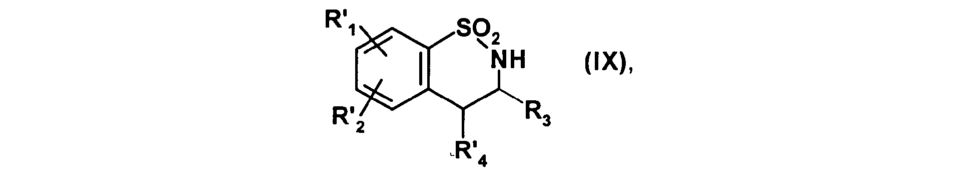 wherein R'₁, R'₂ and R'₄ are as defined hereinbefore,
- **or** the hydroxy group of which is converted to a halogen atom by the action of an appropriate reagent,
to yield the compound of formula (X) : 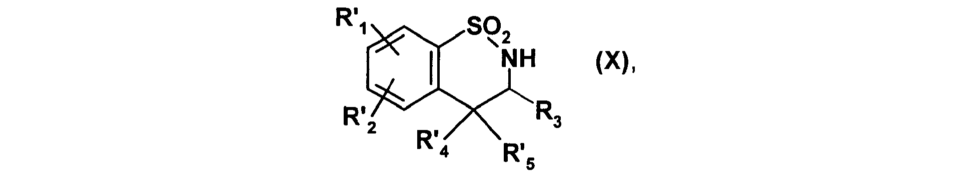
wherein R'₁, R'₂ and R'₄ are as defined hereinbefore and R'₅ represents a halogen atom,
the group R'₁ and the group R'₂, when it represents a linear or branched (C₁-C₆)alkoxy group, of which compounds of formulae (VII) to (X) are converted to hydroxy groups to yield the compound of formula (I/a), a particular case of the compounds of formula (I) : 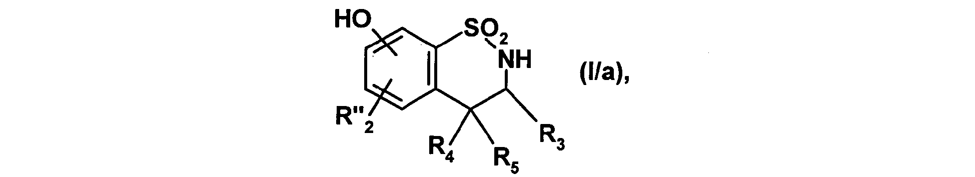 wherein R₄ and R₅ are as defined for formula (I) and R"₂ represents a hydrogen atom, a halogen atom or a hydroxy group,
the hydroxy function or functions of which compound of formula (I/a) are, if desired, converted to corresponding esters,
which compound of formula (I/a) and its corresponding esters, which constitute the totality of the compounds of formula (I),
are purified, if necessary, according to a conventional purification technique, are optionally separated into the isomers according to a conventional separation technique and are converted, if desired, into their addition salts with a pharmaceutically acceptable acid or base.

10. Process for the preparation of compounds of formula (I) wherein A represents an NR₄ group is **characterised in that** there is used as starting material a compound of formula (XI) : 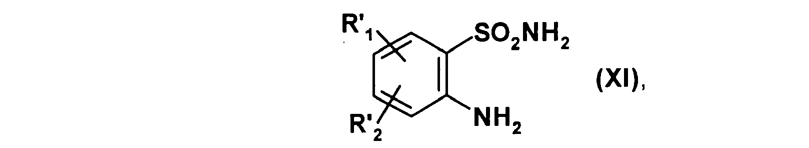 wherein :
R'₁ represents a linear or branched (C₁-C₆)alkoxy group,
R'₂ represents a hydrogen atom, a halogen atom or a linear or branched (C₁-C₆)alkoxy group,
which is cyclised :
- **either** in the presence of an amidine of formula (XII) : 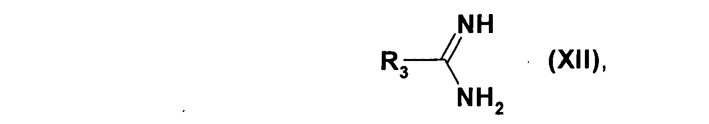 wherein :
R₃ is as defined for formula (I),
to yield the compound of formula (XIII) : 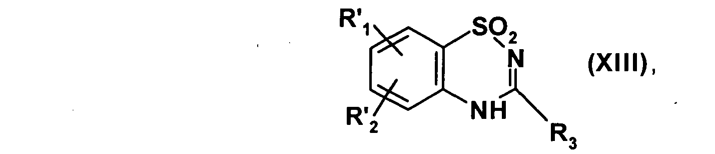 wherein R'₁, R'₂ and R'₃ are as defined hereinbefore,
which is :
◆ either reduced with a metallic hydride
to yield the compound of formula (XIV) : 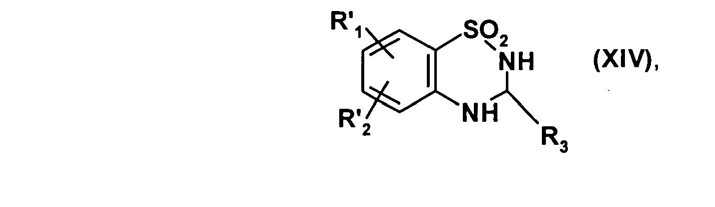 wherein R'₁, R'₂ and R'₃ are as defined hereinbefore,
◆ or alkylated by the action of a strong base in the presence of an alkylating agent R'₄X wherein R'₄ represents a linear or branched (C₁-C₆)alkyl group and X represents a halogen atom, and then reduced
to yield the compound of formula (XV) : 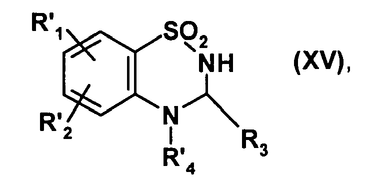
wherein R'₁, R'₂, R₃ and R'₄ are as defined hereinbefore,
- **or** cyclised in the presence of an aldehyde of formula (XVI) : 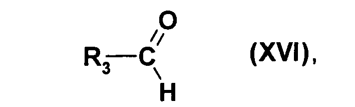
wherein R₃ is as defined for formula (I),
to yield the compound of formula (XIV) described above,
the group R'₁ and the group R'₂, when it represents a linear or branched (C₁-C₆)alkoxy group, of which compound of formula (XIV) or (XV) are converted to hydroxy groups to yield the compound of formula (I/b), a particular case of the compounds of formula (I) : 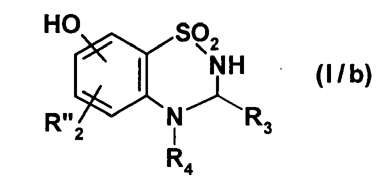 wherein R₃ and R₄ are as defined for formula (I) and R"₂ represents a hydrogen atom, a halogen atom or a hydroxy group,
the hydroxy function or functions of which compound of formula (I/b) are, if desired, converted to corresponding esters,
which compound of formula (I/b) and its corresponding esters, which constitute the totality of the compounds of formula (I),
are purified, if necessary, according to a conventional purification technique, are optionally separated into the isomers according to a conventional separation technique and are converted, if desired, into their addition salts with a pharmaceutically acceptable acid or base.

11. Pharmaceutical compositions comprising as active ingredient a compound according to any one of claims 1 to 8 in combination with one or more pharmaceutically acceptable, inert, non-toxic excipients or carriers.

12. Pharmaceutical compositions according to claim 11 containing as active ingredient a compound according to any one of claims 1 to 8 for use as medicaments, such as AMPA modulators.

## Patentansprüche

1. Verbindungen der Formel (I): 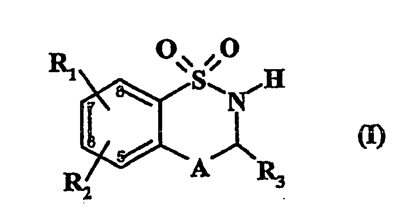 in der:
**R₁** eine Hydroxygruppe oder eine Gruppe RCO-O- bedeutet,
**R₂** ein Wasserstoffatom, ein Halogenatom, eine Hydroxygruppe oder eine Gruppe R'CO-O bedeutet,
**R und R',** die gleichartig oder verschieden sind, eine geradkettige oder verzweigte, gegebenenfalls durch eine Arylgruppe substituierte (C₁-C₆)-Alkylgruppe, geradkettige oder verzweigte, gegebenenfalls durch eine Arylgruppe substituierte (C₂-C₆)-Alkenylgruppe, geradkettige oder verzweigte (C₁-C₆)-Perhalogenalkylgruppe, (C₃-C₇)-Cycloalkylgruppe, Adamantylgruppe, Arylgruppe oder Heteroarylgruppe bedeuten,
**R₃** ein Wasserstoffatom, eine geradkettige oder verzweigte (C₁-C₆)-Alkylgruppe oder eine (C₃-C₇)-Cycloalkylgruppe bedeutet,
**A** eine Gruppe CR₄R₅ oder eine Gruppe NR₄ bedeutet,
**R₄** ein Wasserstoffatom oder eine geradkettige oder verzweigte (C₁-C₆)-Alkylgruppe bedeutet,
**R₅** ein Wasserstoffatom oder ein Halogenatom bedeutet,
deren Isomere sowie deren Additionssalze mit einer pharmazeutisch annehmbaren Säure oder Base,
mit der Maßgabe, dass:
◆ R₁ eine Gruppe RCO-O- bedeutet, wenn sie in der 5-Stellung der Verbindung der Formel (I) steht,
◆ man unter einer Arylgruppe eine aromatische monocyclische Gruppe oder eine bicyclische Gruppe, bei der mindestens einer der Ringe aromatisch ist, versteht, die gegebenenfalls substituiert ist durch ein oder mehrere gleichartige oder verschiedenartige Halogenatome, geradkettige oder verzweigte (C₁-C₆)-Alkylgruppen, geradkettige oder verzweigte (C₁-C₆)-Alkoxygruppen, geradkettige oder verzweigte (C₁-C₆)-Perhalogenalkylgruppen, geradkettige oder verzweigte (C₁-C₆)-Perhalogenalkoxygruppen, Hydroxygruppen, Cyanogruppen, Nitrogruppen, Aminogruppen (die gegebenenfalls durch eine oder mehrere geradkettige oder verzweigte (C₁-C₆)-Alkylgruppen substituiert sind), Aminosulfonylgruppen (die gegebenenfalls durch eine oder mehrere geradkettige oder verzweigte (C₁-C₆)-Alkylgruppen substituiert sind) oder Phenylgruppen (die gegebenenfalls durch eine oder mehrere gleichartige oder verschiedene Halogenatome, geradkettige oder verzweigte (C₁-C₆)-Alkylgruppen, geradkettige oder verzweigte (C₁-C₆)-Perhalogenalkylgruppen, Hydroxygruppen oder geradkettige oder verzweigte (C₁-C₆)-Alkoxygruppen substituiert sind),
◆ man unter einer Heteroarylgruppe eine aromatische monocyclische Gruppe oder eine bicyclische Gruppe, bei der mindestens einer der Ringe aromatisch ist, und die ein, zwei oder drei gleichartige oder verschiedenartige Heteroatome ausgewählt aus Stickstoff, Sauerstoff und Schwefel enthält, versteht, die gegebenenfalls substituiert ist durch ein oder mehrere gleichartige oder verschiedenartige Halogenatome, geradkettige oder verzweigte (C₁-C₆)-Alkylgruppen, geradkettige oder verzweigte (C₁-C₆)-Alkoxygruppen, geradkettige oder verzweigte (C₁-C₆)-Perhalogenalkylgruppen, geradkettige oder verzweigte (C₁-C₆)-Perhalogenalkoxygruppen, Hydroxygruppen, Cyanogruppen, Nitrogruppen, Aminogruppen (die gegebenenfalls durch eine oder mehrere geradkettige oder verzweigte (C₁-C₆)-Alkylgruppen substituiert sind) oder Aminosulfonylgruppen (die gegebenenfalls durch eine oder mehrere geradkettige oder verzweigte (C₁-C₆)-Alkylgruppen substituiert sind).

2. Verbindungen der Formel (I) nach Anspruch 1, **dadurch gekennzeichnet, dass** A eine Gruppe CR₄R₅ bedeutet.

3. Verbindungen der Formel (I) nach Anspruch 1, **dadurch gekennzeichnet, dass** A eine Gruppe NR₄ bedeutet.

4. Verbindungen der Formel (I) nach einem der Ansprüche 1 bis 3, **dadurch gekennzeichnet, dass** die Gruppe R₁ in der 7-Stellung der Verbindung der Formel (I) steht.

5. Verbindungen der Formel (I) nach einem der Ansprüche 1 bis 4, **dadurch gekennzeichnet, dass** R₁ eine Hydroxygruppe bedeutet.

6. Verbindungen der Formel (I) nach einem der Ansprüche 1 bis 4, **dadurch gekennzeichnet, dass** R₁ eine Gruppe RCO-O- bedeutet.

7. Verbindungen der Formel (I) nach einem der Ansprüche 1 bis 6, **dadurch gekennzeichnet, dass** R₂ ein Wasserstoffatom bedeutet.

8. Verbindung der Formel (I) nach Anspruch 1, nämlich 1,1-Dioxido-3,4-dihydro-2*H*-1,2-benzothiazin-7-yl-benzoat.

9. Verfahren zur Herstellung der Verbindungen der Formel (I), in der A eine Gruppe CR₄R₅ bedeutet, **dadurch gekennzeichnet, dass** man als Ausgangsprodukt eine Verbindung der Formel (II) verwendet: 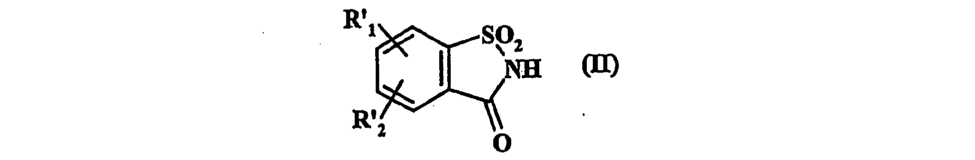 in der:
R'₁ eine geradkettige oder verzweigte (C₁-C₆)-Alkoxygruppe bedeutet und
R'₂ ein Wasserstoffatom. Halogenatom oder eine geradkettige oder verzweigte (C₁-C₆)-Alkoxygruppe darstellt,
welche man der Einwirkung von Chloraceton in Gegenwart einer anorganischen Base in Dimethylformamid-Medium unterwirft zur Bildung der Verbindung der Formel (III): 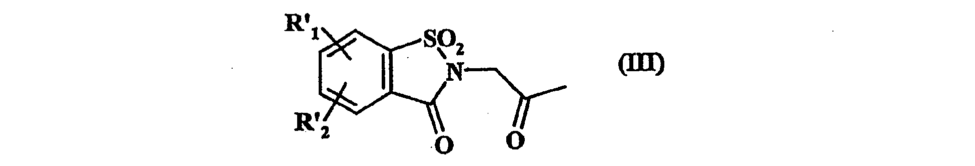 in der R'₁ und R'₂ die oben angegebenen Bedeutungen besitzen,
welche man einer Umlagerung in basischem Medium unterwirft zur Bildung der Verbindung der Formel (IV): 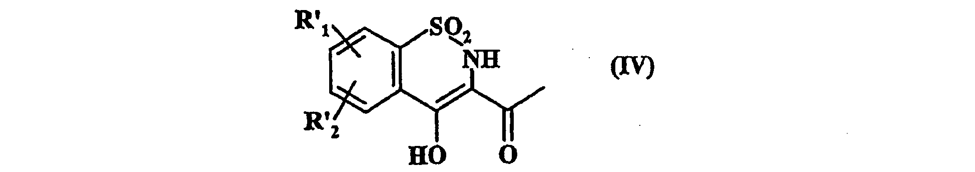 in der R'₁ und R'₂ die oben angegebenen Bedeutungen besitzen,
welche durch Erhitzen zum Sieden am Rückfluss in Benzolmedium in Gegenwart eines Überschusses von Ethylenglykol und einer katalytischen Menge p-Toluolsulfonsäure desacetyliert wird zur Bildung der Verbindung der Formel (V): 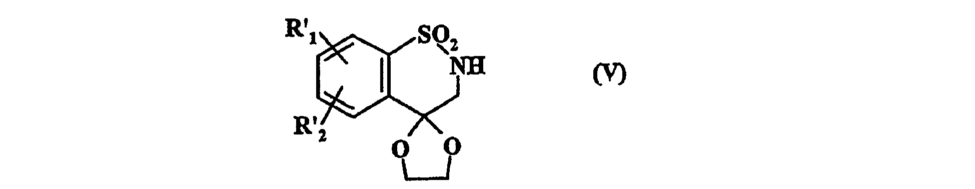 in der R'₁ und R'₂ die oben angegebenen Bedeutungen besitzen,
welche man in saurem Medium hydrolysiert zur Bildung der Verbindung der Formel (VIa): 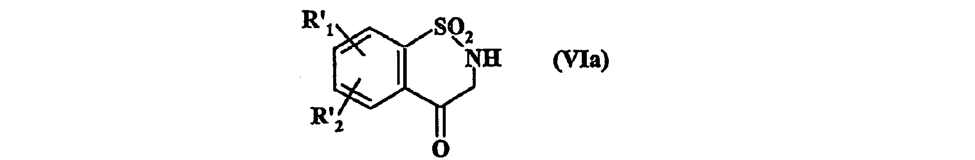 in der R'₁ und R'₂ die oben angegebenen Bedeutungen besitzen,
bei der man gegebenenfalls in dem besonderen Fall, da R₃ von einem Wasserstoffatom verschieden ist, das Stickstoffatom durch eine Schutzgruppe schützt und dann nach der Behandlung mit einer starken Base mit einer Verbindung der Formel R'₃-P behandelt,
in der R'₃ eine geradkettige oder verzweigte (C₁-C₆)-Alkylgruppe oder eine (C₃-C₇)-Cycloalkylgruppe und P eine austretende Gruppe bedeuten,
so dass man nach der Abspaltung der Schutzgruppe des Stickstoffatoms die Verbindung der Formel (VI'a) erhält: 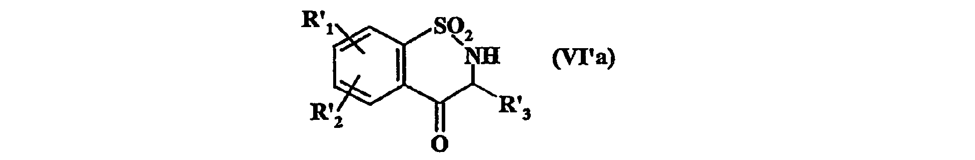 in der R'₁, R'₂ und R'₃ die oben angegebenen Bedeutungen besitzen,
welche Verbindungen der Formeln (VIa) und (VI'a), die durch die Formel (VI) wiedergegeben werden: 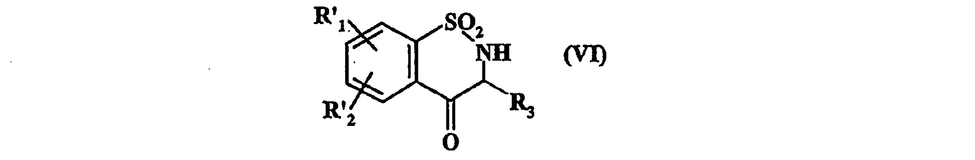 in der R'₁ und R'₂ die oben angegebenen Bedeutungen besitzen und R₃ die bezüglich der Formel (I) angegebenen Bedeutungen besitzt,
man:
- **entweder** einer katalytischen Reduktion unterzieht zur Bildung der Verbindung der Formel (VII): 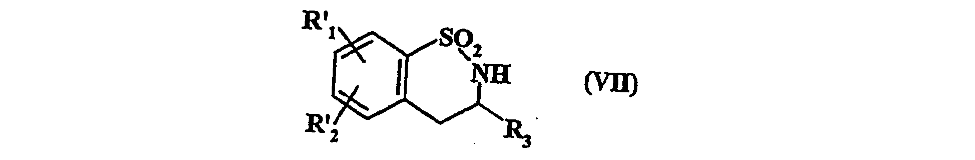 in der R'₁ und R'₂ die oben angegebenen Bedeutungen besitzen,
- **oder** durch Einwirkung eines Hydrids in den Alkohol überführt, dessen Hydroxygruppe man durch Einwirkung eines geeigneten Reagenz in ein Halogenatom umwandelt,
zur Bildung der Verbindung der Formel (VIII): 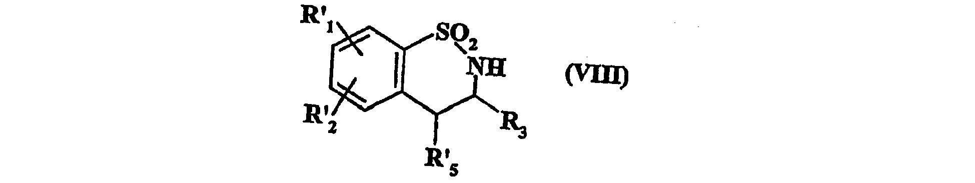 in der R'₁ und R'₂ die oben angegebenen Bedeutungen besitzen und R'₅ ein Halogenatom bedeutet,
- **oder** der Einwirkung einer magnesiumorganischen Verbindung R'₄ MgBr, worin R'₄ eine geradkettige oder verzweigte (C₁-C₆)-Alkylgruppe darstellt, unterwirft,
zur Bildung der Verbindung der Formel (VIb): 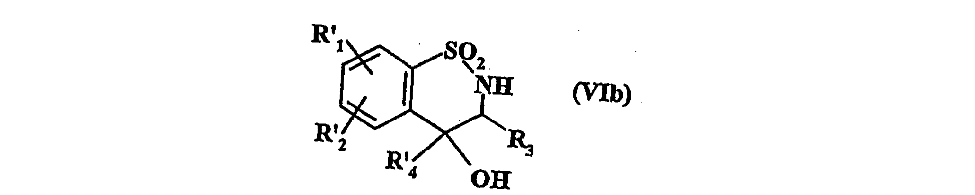 in der R'₁, R'₂ und R'₄ die oben angegebenen Bedeutungen besitzen,
welche Verbindung der Formel (VIb):
- **entweder** einer katalytischen Reduktion unterworfen wird zur Bildung der Verbindung der Formel (IX): 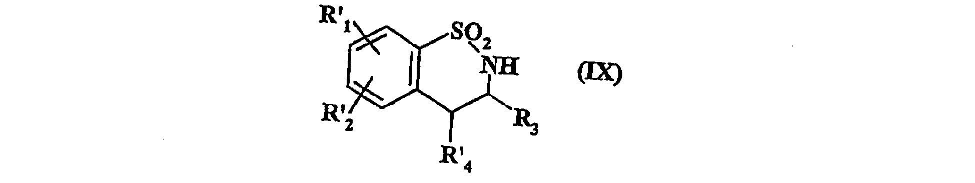 in der R'₁, R'₂ und R'₄ die oben angegebenen Bedeutungen besitzen,
- **oder** bei der eine Hydroxygruppe durch Einwirkung eines geeigneten Reagens in ein Halogenatom umgewandelt wird,
zur Bildung der Verbindung der Formel (X): 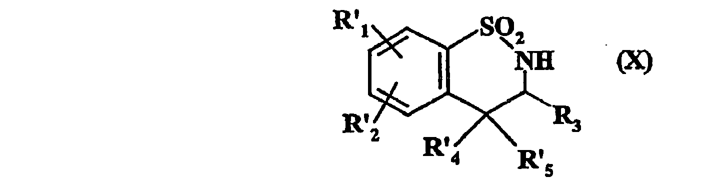 in der R'₁, R'₂ und R'₄ die oben angegebenen Bedeutungen besitzen und R'₅ ein Halogenatom darstellt,
von welchen Verbindungen der Formeln (VII) bis (X) man die Gruppe R'₁ und die Gruppe R'₂, wenn diese eine geradkettige oder verzweigte (C₁-C₆)-Alkoxygruppe darstellt, in Hydroxygruppen umwandelt,
zur Bildung der Verbindung der Formel (I/a), einem Sonderfall der Verbindungen der Formel (I): 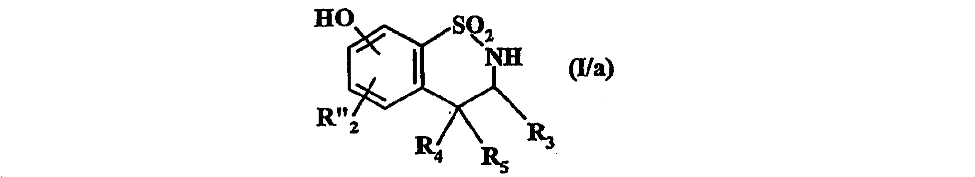 in der R₄ und R₅ die bezüglich der Formel (I) angegebenen Bedeutungen besitzen und R"₂ ein Wasserstoffatom, ein Halogenatom oder eine Hydroxygruppe bedeutet, von welchen Verbindungen der Formel (I/a) man gewünschtenfalls die Hydroxy-funktion(en) in den entsprechenden Ester umwandelt,
welche Verbindungen der Formel (I/a) und ihre entsprechenden Ester die Gesamtheit der Verbindungen der Formel (I) bilden,
welche man gegebenenfalls mit Hilfe einer klassischen Reinigungsmethode reinigt, welche man gegebenenfalls mit Hilfe einer klassischen Trennmethode in ihre Isomeren auftrennt und welche man gewünschtenfalls in ihre Additionssalze mit einer pharmazeutisch annehmbaren Säure oder Base umwandelt.

10. Verfahren zur Herstellung der Verbindungen der Formel (I), in der A eine Gruppe NR₄ bedeutet, **dadurch gekennzeichnet, dass** man als Ausgangsprodukt eine Verbindung der Formel (XI) verwendet: 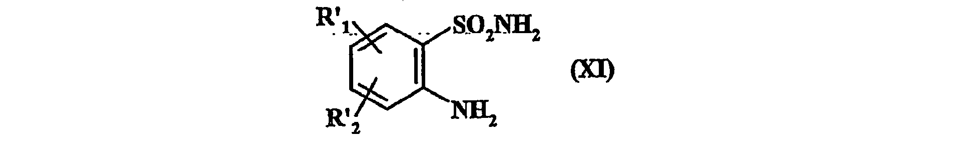 in der:
R'₁ eine geradkettige oder verzweigte (C₁-C₆)-Alkoxygruppe bedeutet und
R'₂ ein Wasserstoffatom, ein Halogenatom oder eine geradkettige oder verzweigte (C₁-C₆)-Alkoxygruppe bedeutet,
welche man:
- **entweder** in Gegenwart eines Amidins der Formel (XII) cyclisiert: 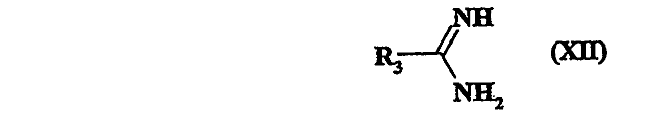 in der:
R₃ die bezüglich der Formel (I) angegebenen Bedeutungen besitzt,
zur Bildung der Verbindung der Formel (XIII): 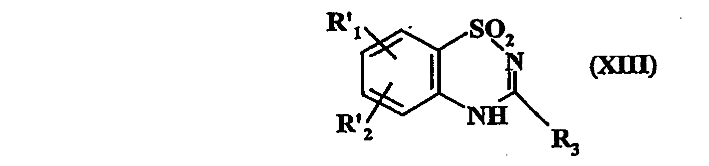 in der R'₁, R'₂ und R'₃ die oben angegebenen Bedeutungen besitzen,
welche:
◆ entweder mit einem Metallhydrid reduziert wird
zur Bildung der Verbindung der Formel (XIV): 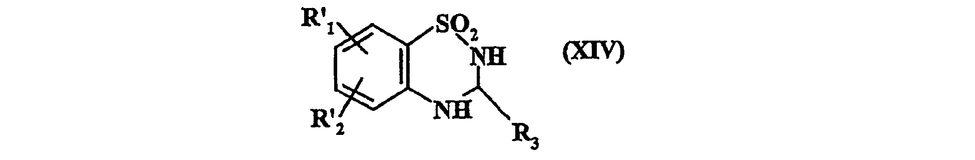 in der R'₁, R'₂ und R'₃ die oben angegebenen Bedeutungen besitzen,
◆ oder durch Einwirkung einer starken Base in Gegenwart eines Alkylierungsmittels R'₄X, worin R'₄ eine geradkettige oder verzweigte (C₁-C₆)-Alkylgruppe und X ein Halogenatom bedeuten, alkyliert und dann reduziert wird, zur Bildung der Verbindung der Formel (XV): 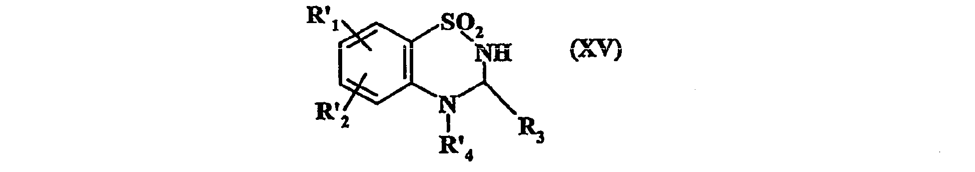 in der R'₁, R'₂, R'₃ und R'₄ die oben angegebenen Bedeutungen besitzen,
- **oder** in Gegenwart eines Aldehyds der Formel (XVI) cyclisiert wird: 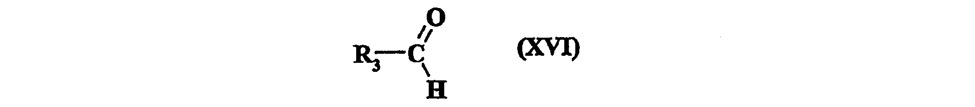 in der R₃ die bezüglich der Formel (I) angegebenen Bedeutungen besitzt,
zur Bildung der Verbindung der oben beschriebenen Formel (XIV),
von welchen Verbindungen der Formeln (XIV) bis (XV)
man die Gruppe R'₁ und die Gruppe R'₂, wenn diese eine geradkettige oder verzweigte (C₁-C₆)-Alkoxygruppe bedeuten, in Hydroxygruppen umwandelt,
zur Bildung der Verbindung der Formel (I/b), einem Sonderfall der Verbindungen der Formel (I): 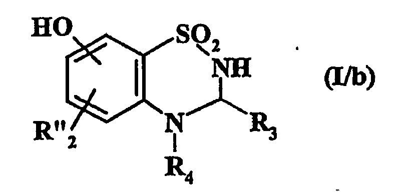 in der R₃ und R₄ die bezüglich der Formel (I) angegebenen Bedeutungen besitzen und R"₂ ein Wasserstoffatom, ein Halogenatom oder eine Hydroxygruppe darstellt,
von welchen Verbindungen der Formel (I/b) man gewünschtenfalls die Hydroxy-funktion(en) in die entsprechenden Ester umwandelt,
welche Verbindungen der Formel (I/b) und ihre entsprechenden Ester die Gesamtheit der Verbindungen der Formel (I) bilden,
welche man gegebenenfalls mit Hilfe einer klassischen Reinigungsmethode reinigt, man gegebenenfalls mit Hilfe einer klassischen Trennmethode in ihre Isomeren auftrennt und gewünschtenfalls in ihre Additionssalze mit einer pharmazeutisch annehmbaren Säure oder Base umwandelt.

11. Pharmazeutische Zubereitungen enthaltend als Wirkstoff eine Verbindung nach einem der Ansprüche 1 bis 8 in Kombination mit einem oder mehreren inerten, nichttoxischen, pharmazeutisch annehmbaren Trägermaterialien oder Hilfsstoffen.

12. Pharmazeutische Zubereitungen nach Anspruch 11, enthaltend als Wirkstoff eine Verbindung nach einem der Ansprüche 1 bis 8 nützlich als Arzneimittel, namentlich als AMPA-Modulatoren.
